(19) Europäisches Patentamt
European Patent Office
Office européen des brevets

(11) Publication number: **0 113 508**
**A1**

# (12) EUROPEAN PATENT APPLICATION

(21) Application number: **83306726.7**

(22) Date of filing: **04.11.83**

(51) Int. Cl.[3]: **C 07 F 15/00**
A 61 K 9/50, A 61 K 31/685

(30) Priority: **04.11.82 US 439132**
**04.08.83 US 520468**

(43) Date of publication of application:
**18.07.84 Bulletin 84/29**

(84) Designated Contracting States:
**BE DE FR GB IT NL**

(71) Applicant: **Inco Research & Development Center, Inc.**
**Sterling Forest**
**Suffern New York 10901(US)**

(72) Inventor: **Sears, Barry**
**281 Albany Street**
**Cambridge, MA 02139(US)**

(74) Representative: **Greenstreet, Cyril Henry et al,**
**Thames House (Fifth floor) Millbank**
**London SW1P 4QF(GB)**

## (54) Hydrophobic platinum compounds and their preparation.

(57) Novel *cis*-platinum (II) compounds having amino and phosphatidic acid substituents, which may be incorporated in a phospholipid vesicle for therapeutic treatment of mammals, and their preparation.

EP 0 113 508 A1

# Hydrophobic platinum compounds and their preparation

Certain platinum compounds, and particularly cis platinous compounds, have demonstrated antitumour activity in mammals. The literature pertaining to these compounds and their activity is extensive. Among patents which can be mentioned, each of which discusses earlier art, are U.S. Patents Nos. 4 140 707, 4 177 263, 4 202 890 and 4 207 416. It is also known that the cis platinum (II) compounds are toxic.

Workers in the art have made many attempts to employ the useful properties of the cis Pt (II) compounds while, at the same time, reducing their toxic effects. Thus, not only can the compounds be lethal, but smaller dosages can cause nausea and other undesirable reactions. The art has in the main sought to employ platinous compounds which are soluble.

The art has also envisioned the use of delivery systems of various kinds in endeavours to improve the selectivity of drugs used for the treatment of various mammalian ailments. Liposomes as carriers for biological substances have been discussed in the literature for a number of years, as illustrated by the article "The Carrier Potential of Liposomes in Biology and Medicine" by Gregoriadis which appeared in The New England Journal of Medicine for September 23, 1976 at page 704 et seq. Gregoriadis reviewed many specified promising effects but concluded that "where the cellular interior is to be reached, persuading the cell to allow passage to the carrier may need Odyssean wiliness".

The invention is directed to new cis Pt (II) compounds which in contrast to compounds heretofor used as antitumour agents, have little or no solubility in the plasma and which preferably are employed with a lipid vesicle carrier. It also relates to the preparation of the compounds and to their use in particular when encapsulated in a lipid carrier of controlled diameter,

for physiological purposes in mammals.

The cis Pt (II) compounds of the invention have the general formula:

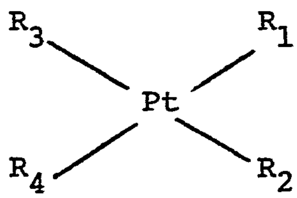

wherein $R_1$ and $R_2$ are selected from the group $NH_3$; 1,2-diaminocyclohexanyl (including each and every isomer thereof and mixtures of said isomers); cyclohexylamine; cyclopentylaminyl and isoamylaminyl, with the proviso in the case of 1,2-diaminocyclohexanyl group that the ligands represented by $R_1$ and $R_2$ can be the two amine groups on a single diaminocyclohexane molecule, i.e. a bridging of $R_1$ and $R_2$;

$R_3$ is a phosphatidyl group (PG) having fatty acid substituents containing 12 to 20 carbon atoms and which may be saturated or unsaturated; and

$R_4$ is a biologically compatible negatively charged ion, particularly ions selected from the group PG, $NO_3$ and Cl.

In the compounds, $R_1$ and $R_2$ are considered to be covalently bonded, while $R_3$ and $R_4$ are considered to be ionically bonded. The term "cis" when used in this specification and claims relates to the adjacent positions of covalently bonded $R_1$ and $R_2$ in the described and defined compounds and not to the ultimate optical rotary power of the total compound or to the position of any other isomeriferous grouping in the total compound. For example, the diaminocyclohexane (DAC) used in preparing compounds of the present invention was a technical grade of material sold by Aldrich Chemical Co. containing about 85% of diaminocyclohexane isomers including cis and trans isomers and plus and minus varieties of the transisomer. Thus the term

*cis* when used in this specification and claims relating to DAC complexed materials has no significance relative to the DAC portion of the total molecule.

In general, the compounds of the invention can be produced by reacting a donor including a phosphatidyl group derived from a phospholipid and having a net negative charge with a dinitrato *cis* Pt(II) compound in a liquid reaction medium in which both reactants are soluble to interchange at least one phosphatidyl group for one nitrate group and precipitate the resulting compound from the reaction medium.

Compounds of the invention may be prepared for example by converting a phosphatidic acid to salt form, dissolving the salt in a solvent having suitable dielectric strength and mixing a *cis* platinum (II) salt, e.g. a nitrate or aquated nitrate salt, with the solution to form the corresponding *cis* platinum (II) phosphatidic acid analogue. The analogue may then be incorporated in a unilamellar phospholipid vesicle or multilammellar liposome. Phosphatidic acid can be produced in a known manner, e.g. by enzymatic action on a phosphatidyl choline.

Features of the invention which facilitate production of the compounds of the invention include conversion of the phosphatidic acid to salt form (e.g. ammonium form) rather than the protonated form, so as to permit ligand displacement and control of the dielectric strength of the reaction medium. When the protonated form of phosphatidic acid is used, no reaction takes place unless a large excess of the phosphatidic acid is present.

The reaction medium may be methanol or a mixture of methanol and chloroform to which water is added to raise its dielectric constant. The water content of the reaction medium must be high enough

to raise its dielectric constant and to obtain good yield of product, but not so high as to precipitate the phosphatidic acid or its salt. It appears that optimal yields result through use of a reaction medium containing methanol to which water is added in the maximum amount which the reaction medium will tolerate and still remain a single phase. In most instances this is approximately 17% water, by volume.

As is known, phosphatidylcholine is a term applied to compounds which are esters of fatty acids with glycerophosphoric acid and choline. These compounds are commercially available in substantially pure form by isolation from natural sources such as soybeans or egg yolk. Other phosphatidylcholines may be synthesized by the procedure such as described by Robles and van der Berg, Biochim Biophys Acta, 187:520 (1969).

The cis platinum (II) phosphatidic analogues provided in accordance with the invention are insoluble in water and cannot be dispersed by sonication. In order to incorporate an analogue in a phosphatidylcholine vesicle, e.g. soy phosphatidylcholine, it is necessary to take the mixture of the ingredients to dryness and lyophilize it, e.g. from cyclohexane. The resulting freeze-dried material is easily hydrated and can be sonicated with ease to produce small sonicated vesicles which have high stability in physiological saline.

Some examples will now be given.

Example 1

Dipalmitoyl phosphatidylcholine and dioleyl phosphatidylcholine were synthesized by standard procedures. Egg phosphatidylcholine and soy phosphatidylcholine were purified from natural sources. The corresponding phosphatidic acid was synthesized by the digestion of the appropriate phosphatidylcholine using phospholipase D obtained from Savoy cabbage. The

phosphatidic acid was converted to the ammonium salt form by passing through an ion exchange resin bed (the resin being in the ammonium salt form) using 1:1 chloroform/methanol as the eluting solvent.

The general method of the synthesis of the cis Pt (II) compounds was as follows: As a starting material a cis Pt (II) compound of the following formula was provided:

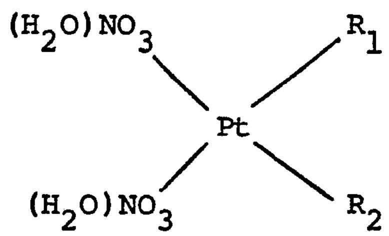

The cis configuration of the nitrato compounds is assured by preparation of these compounds through the route of

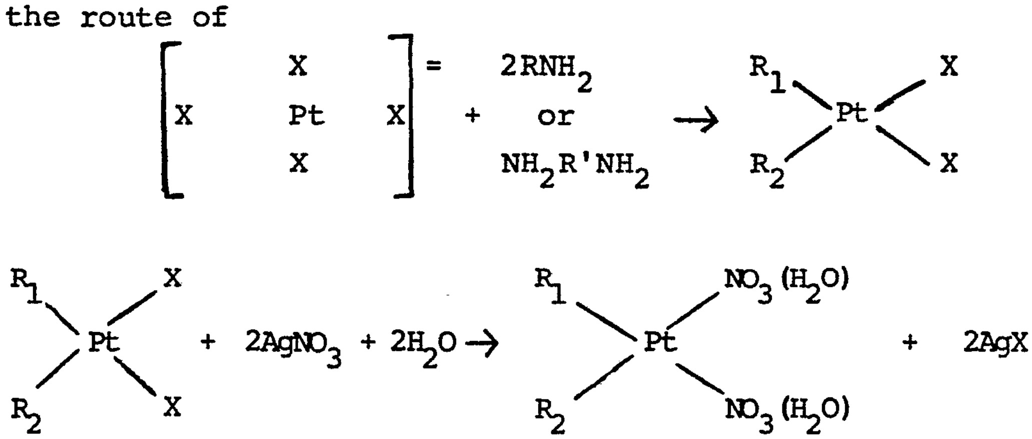

wherein X = Cl or I, R is hydrogen or an aliphatic or cycloaliphatic group, R' is an alkylene group or a cycloalkylene group, $R_1$ and $R_2$ represent ammine ligands and $NO_3(H_2O)$ represents the aquated, nitrato functionality regardless of which entity $NO_3$ or $H_2O$ is directly complexed in the planar ring surrounding the platinum atom. The cis configuration of these nitrato compounds is assured by this synthetic route by virtue of the greater trans-directing influence of the halide

compared to the trans-directing influence of $NH_3$ or an aliphatic or cycloaliphatic primary amine or diamine. The cis configuration of certain ones of the nitrato compounds used in examples set forth herein has been confirmed by use of the Kurnakov test as described in Inorganic Synthesis, Vol. 7, J. Kleinberg, 1963, page 244.

The cis Pt (II) starting dinitrato material is neutralized with 0.5 M $NH_4OH$ to approximately pH 4.5. The water from the sample was evaporated under vacuum at temperature less than 30°C. The dried dinitrato cis Pt (II) compound was then taken up in methanol. (Methanol used in all the preparations described herein is absolute methanol). To form the desired phosphaditic acid cis Pt (II) compound, two moles of phosphaditic ammonium salt in 88% (by volume) $CHCl_3$,balance methanol, at a concentration of about 100 μ mole per ml were added to one mole of the dinitrato cis Pt (II) compound dissolved in methanol at a concentration approaching that of saturation. Water was added to the reaction medium to increase the dielectric constant, but yet maintain a single phase system. Samples were stirred at room temperatures under a nitrogen atmosphere and then kept overnight at 4°C. A precipitated oil or a flocculant precipitate (depending on the degree of unsaturation of the phosphaditic acid) collected at the bottom of the reaction flask. The clear supernatant was decanted. The precipitate was dissolved in a minimal volume of methylene chloride and sufficient methanol was added to reprecipitate the phosphaditic acid cis Pt (II) compound. Such repurification was continued until the phosphate/platinum ratio of the complex was 1.3 or less. The compounds were dissolved in methylene chloride and stored at -20°C.

## Example 2

## Synthesis of Dipalmitoyl Phosphatidic Acid 1,2 Diaminocyclohexane Cis Pt (II)

5227 μ moles of the ammonium salt of dipalmitoyl phosphaditic acid in chloroform/methanol were taken to dryness. The compound was dissolved in 400 ml of methanol to give a clear solution. To this solution was added 2376 μ moles of dinitrato 1,2 diaminocyclohexane cis Pt (II) in methanol. Water was added to the limit of a single phase and to maintain solubility of the reactants. The reaction mixture was stirred for two hours at room temperature, and then placed overnight at 4°C. The clear supernatant was decanted from a white precipitate. The initial yield of Pt in the precipitate was 95%. The phosphate to platinum ratio in the precipitate was 1.64. After one reprecipitation from methanol, the phosphate to platinum ratio was reduced to 1.26, and after a second reprecipitation the ratio was further reduced to 1.13.

## Example 3

## Synthesis of Dioleyl Phosphaditic Acid 1,2 Diaminocyclohexane Cis Pt (II)

5633 μ moles of dioleyl phosphaditic acid as the ammonium salt in methanol/chloroform were taken to dryness. 250 ml of methanol were added to the dried material. To this solution was added a solution of 2564 μ moles of dinitrato 1,2 diaminocyclohexane cis Pt (II) over a 5-minute period with stirring. Water was added as in Example 2. Within minutes a green oil began to deposit on the walls of the reaction flask. The solution was allowed to sit overnight at 4°C. The clear supernatant was decanted from the green precipitated oil. The yield of the compound was 71% based on the original platinum added. The ratio of phosphate to platinum was 1.19.

Example 4

Synthesis of Egg Phosphaditic Acid 1,2 Diaminocyclohexane Cis Pt (II)

To a solution of 4356 μ moles of egg phosphaditic acid in the ammonium salt form in methanol was added 1980 μ moles of dinitrato 1,2 diaminocyclohexane cis Pt (II) in methanol over a 5 minute period. Water was added as in Example 2. A green precipitate gradually formed. The solution was stirred for 2 hours at room temperature and then stored overnight at 4°C. The yield of the compound was 89% based upon platinum added. The ratio of phosphate to platinum was 1.21.

Example 5

Synthesis of Soy Phosphaditic Acid Bis Cyclohexylamine Cis Pt (II)

1800 μ moles of soy phosphatidic acid in the ammonium salt form were dissolved in 10 ml of methylene chloride and 60 ml of methanol. To this stirred solution were added 900 μ moles of dinitrato bis-cyclohexylamine cis Pt (II) in 65 ml of methanol. Over a period of 3 hours a total of 17 ml of water was added gradually to the stirred solution at room temperature. After 5 hours total at room temperature, the solution was then stored at -10°C overnight. The supernatant was decanted and the yield of the platinum compound based upon platinum added was 75%. The phosphate to platinum ratio of the precipitate was 1.76. The precipitate was dissolved in a minimal amount of methylene chloride and then methanol was added until a slight cloudiness appeared in the solution at room temperature. The solution was then cooled overnight at 4°C. The precipitate was recovered and the procedure was repeated once more. The phosphate to platinum ratio after the second precipitation was 1.32.

Example 6

Synthesis of Soy Phosphatidic Acid Bis Cyclopentylamine Cis Pt (II)

1800 μ moles of soy phosphaditic acid in the ammonium salt form were dissolved in 10 ml of methylene chloride and 60 ml of methanol. To a stirred solution was added 900 μ moles of dinitrato bis-cyclopentylamine cis Pt (II) in 28 ml of methanol. An additional 70 ml of methanol and 10 ml of methylene chloride was added to the solution. Over a 3 hour period 17 ml of water were added to the stirred solution at room temperature. After 5 hours total at room temperature, the solution was stored at -10°C. The supernatant was decanted and the yield of platinum in the precipitate was 97%. The phosphate to platinum ratio was 1.64. After three reprecipitations from methylene chloride/methanol mixtures the phosphate to platinum ratio had decreased to 1.28.

Example 7

Synthesis of Soy Phosphatidic Acid Bis Isoamylamine Cis Pt (II)

1446 μ moles of soy phosphatidic acid in the ammonium salt form were dissolved in chloroform. 723 μ moles of dinitrato bis-isoamylamine cis Pt (II) in 68 ml methanol was added to the stirred solution. After 2 hours, 4 ml of water was added, and the solution was stored at 4°C. The clear supernatant was decanted, and the yield of platinum in the precipitate was 50%. The phosphate to platinum ratio of the precipitate was 1.37. No further purification via methylene chloride/methanol precipitation was attempted on this compound.

One key step to the success of the above synthesis was the use of the salt form, particularly the ammonium salt form, of phosphaditic acid. If the protonated form of phosphatidic acid was used, essentially no reaction took place under the experimental conditions of Examples 2 to 7. A second key aspect is the stoichiometry of the product. The reaction conditions are such that any phosphatidic acid not complexed to the platinum compound may also precipitate. The phosphatidic to platinum stoichiometric ratios of all of the initial crude precipitates were less than 2.0, indicating that the actual compound may have only one phosphaditic acid complexed to the platinum. Purification by absorption chromatography caused extensive displacement of the phosphatidic acid from the complex. Gel permeation chromatography using LH-20 gave poor results. Therefore, reprecipitations from methylene chloride (in which the complex is very soluble) and methanol (in which the complex is relatively insoluble compared to phosphatidic acid) were employed to achieve partial purification of the compound. This is important in two respects: first, to obtain as pure a compound as possible, and second, to facilitate incorporation of the compound into a sonicated phosphatidylcholine vesicle. It has been found that greater amounts of free phosphatidic acid present in the vesicle in addition to the cis Pt (II) compound decreases the overall structural stability of the vesicle. This is in direct contrast to the stabilizing effect of phosphatidic acid found in the case of similar sonicated vesicles in the absence of cis Pt (II) analogues.

## Example 8

## Preparation of Cis Pt (II) Analogue in Phosphatidylcholine Vesicles

To deliver the water insoluble cis Pt (II) analogues for chemotherapy, they were encapsulated in sonicated phosphatidylcholine (PC) vesicles. To prepare these PC vesicles, both the cis Pt (II) analogue and PC had to be lyophilized from cyclohexane or benzene to give a dry powder for hydration by an aqueous medium. Simply drying down an organic solution of both the cis Pt (II) analogue and the PC gave unsatisfactory

results upon hydration. The lyophilized samples were hydrated with physiological saline and then sonicated at 4°C using a W-375 Branson sonicator, under a nitrogen atmosphere. The sonicated samples were spun at 12,000 x G for 5 minutes to remove any titanium fragments from the sonicator probe and any undispersed lipid. Experiments indicated that, even when sonication was performed at 4°C, there was displacement of the phosphatidic acid ligands by chloride ions. Therefore all preparations were passed down a G-50 gel permeation column to separate any free cis Pt (II) from the hydrophobic cis Pt (II) analogue encapsulated in egg PC vesicles had better structural integrity and more uniform size distribution than the same analogues in soy PC vesicles. Furthermore, it was found that the structural stability and size distribution of the vesicles containing the cis Pt (II) analogues were inversely correlated with the amount of free phosphatidic acid content in the vesicle. That is, the vesicles containing the cis Pt (II) analogues that had the lowest amounts of free phosphatidic acid, had the best structural stability and smaller size ranges. This is in direct contrast to the effect of phosphatidic acid in PC vesicles in the absence of the cis Pt (II) analogues.

The fractions from the G-50 column containing the encapsulated cis Pt (II) analogues were concentrated by ultrafiltration to the desired concentration for chemotheraphy studies. Analysis indicated that these samples had no free water soluble cis Pt (II) as indicated by the degree of partition of the platinum in a Folch two-phase system.

Various compounds synthesized and incorporated in liposomal vesicles in the foregoing manner were employed in the chemotherapy of mice. The testing consisted of injections of $10^{-5}$ L1210 leukemic cells intravenuously into mice. One day later, a single

intravenous dose of the cis Pt (II) analogue in a sonicated PC vesicle was given. Increase in life span (ILS) was then measured relative to control animals who received only saline or PC vesicles alone as intravenous injections. Data are shown in Table I, together with data on some tests in which samples were not passed through a gel permeation column to remove free cis Pt (II), but an injectable preparation was made by the addition of "Tween" 80.

As can be seen from Table I, the chemotherapy is highly dependent on the degree of acyl chain saturation of the phosphatidic acid, the nature of the PC used as the liposomal carrier system, and the amino ligand of cis Pt (II) analogue. Furthermore, the toxicity the cis Pt (II) analogues (i.e. $LD_{10}$) appears to have little direct relationship to the observed chemotherapy.

The results indicate that the substituted cis Pt (II) compounds are considerably less toxic than the diaminodichloro cis Pt (II) compound itself. Thus, $LD_{10}$ for cis Pt (II) is about 15 mg Pt/kg under the conditions reported.

The particle size of the sonicated phospholipid vesicles bearing the cis Pt (II) analogues were in the range of about 200 to 500Å.

**TABLE 1**

**PRELIMINARY CHEMOTHERAPY RESULTS**

| Cis Pt(II) Compound | PC Vesicle | % ILS at Optimal Dose | Optimal Dose (mg Pt/kg) | $LD_{10}$ (mg Pt/kg) |
|---|---|---|---|---|
| Dipalmitoyl phosphatidic acid DAC | Egg | 100 | 39 | 58 |
| Dioleyl phosphatidic acid DAC | Egg | 83 | 39 | 58 |
| Egg phosphatidic acid DAC | Egg | 50 | 26 | >58 |
| Soy phosphatidic acid bis cyclohexylamine | Soy | 16 | 155 | 500 |
| Soy phosphatidic acid bis cyclopentylamine | Soy | 16 | 125 | 125 |
| Soy phosphatidic acid bis isoamylamine | Soy | 8 | 155 | 345 |
| Soy phosphatidic acid DAC* | Soy | 83 | 58 | 58 |
| Soy phosphatidic acid diamino* | Soy | 33 | 51 | 51 |
| Dioleyl phosphatidic acid DAC* | Soy | 0 | - | - |

* Samples not treated by G-50 column and Tween-80 added.

DAC = Diaminocyclohexyl bridging ligand derived from a racemic mixture of diastereomers of diaminocyclohexane comprising approximately a 50-50 mixture of cis-trans isomers with the trans material being a mixture of trans-minus and trans-plus. It has not been determined whether or not reaction of this diaminocyclohexane to form the bridging ligand (DAC) is selective with respect to any of these isomeric forms.

Claims

1. A compound of the formula:

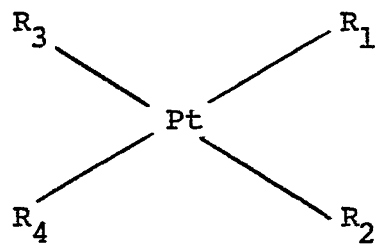

wherein

$R_1$ and $R_2$ are selected from the group $NH_3$, 1,2 diaminocyclohexyl, cyclohexylaminyl, cyclopentylaminyl and isoamylaminyl, with the proviso that $R_1$ and $R_2$ are identical or, in the case of 1, 2 diaminocyclohexyl, $R_1$ and $R_2$ may be satisfied by the amino groups on the same ring to provide a bridging ligand;

$R_3$ is a phosphatidyl group (PG) having a net negative charge and having fatty acid substituents containing from 12 to 20 carbon atoms and which may be saturated or unsaturated; and

$R_4$ is a biologically compatible negatively charged ion.

2. A compound according to claim 1 wherein $R_4$ is PG, $NO_3$ and Cl.

3. A compound in accordance with claim 1 wherein $R_1$ and $R_2$ comprise a 1,2 diaminocyclohexyl bridging ligand.

4. A compound according to claim 1, being
dipalmitoyl phosphatidic acid 1,2 diaminocyclohexane cis Pt(II),
dioleyl phosphatidic acid 1,2 diaminocyclohexane cis Pt (II),
egg phosphatidic acid 1, 2 diaminocyclohexane cis Pt (II),
soy phosphaditic acid bis cyclohexylamine cis Pt (II),
soy phosphaditic acid bis cyclopentylamine cis Pt (II), or
soy phosphaditic acid bis isoamylamine cis Pt (II).

5. A compound according to any preceding claim incorporated in a phospholipid vesicle.

6. A compound according to claim 5 wherein said phospholipid is a phosphatidyl choline.

7. Method for preparing a cis platinum II phosphatidyl analogue according to claim 1 which comprises reacting a phosphatidyl donor with a dinitrato cis platinum II compound in a liquid reaction medium in which both reactants are soluble to interchange at least one phosphatidyl group with one nitrato group and precipitate the resulting cis platinum II phosphatidyl analogue from said liquid medium.

8. Method according to claim 7 wherein the phosphatidyl donor is an ammonium salt of a phosphatidic acid.

9. Method according to claim 6 or claim 7 wherein the dielectric strength of the reaction medium is equivalent to that of methanol containing about 17% water, by volume.

European Patent Office

# EUROPEAN SEARCH REPORT

0113508

Application number

EP 83 30 6726

**DOCUMENTS CONSIDERED TO BE RELEVANT**

| Category | Citation of document with indication, where appropriate, of relevant passages | Relevant to claim | CLASSIFICATION OF THE APPLICATION (Int. Cl. 3) |
|---|---|---|---|
| A | FR-A-2 517 310 (UNILEVER NL)<br>* Page 21, claim 1 *<br>--- | 1 | C 07 F 15/00<br>A 61 K 9/50<br>A 61 K 31/685 |
| A | CHEMICAL ABSTRACTS, vol. 96, no. 10, 8th March 1982, page 374, no. 74589c, Columbus, Ohio, US<br>G.I. MUZYA et al.: "Antitumor and toxic properties of phospholipid vesicles containing cis-dichlorodiaminoplatinum" & BYULL. EKSP. BIOL. MED. 1981, 92(11), 590-3<br>----- | 1 | |
| | | | TECHNICAL FIELDS SEARCHED (Int. Cl. 3) |
| | | | C 07 F 15/00<br>C 07 F 9/00 |

The present search report has been drawn up for all claims

| Place of search | Date of completion of the search | Examiner |
|---|---|---|
| THE HAGUE | 06-03-1984 | SUTER M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document